Europäisches Patentamt

European Patent Office

Office européen des brevets

(11) Veröffentlichungsnummer: **0 421 021 A1**

# (12) EUROPÄISCHE PATENTANMELDUNG

(21) Anmeldenummer: 89118625.6

(22) Anmeldetag: 06.10.89

(51) Int. Cl.5: **A61K 37/54**

(43) Veröffentlichungstag der Anmeldung:
**10.04.91 Patentblatt 91/15**

(84) Benannte Vertragsstaaten:
**AT BE CH DE ES FR GB GR IT LI LU NL SE**

(71) Anmelder: **MUCOS EMULSIONSGESELLSCHAFT m.b.H.**
**Miraustrasse 17**
**W-1000 Berlin 27(DE)**

(72) Erfinder: **Ransberger, Karl**
**Gabriel-von-Seidl-Strasse 62**
**W-8022 Grünwald(DE)**
Erfinder: **Stauder, Gerhard, Dr.**
**Kathi-Kobus-Steig 1**
**W-8190 Wolfratshausen(DE)**

(74) Vertreter: **Patentanwälte Grünecker,**
**Kinkeldey, Stockmair & Partner**
**Maximilianstrasse 58**
**W-8000 München 22(DE)**

(54) Verwendung katabolischer Enzyme zur Aktivierung von Makrophagen und/oder NK-Zellen, ein diese Enzyme enthaltendes Arzneimittel sowie durch diese Enzyme aktivierte Makrophage und/oder NK-zelle.

(57) Die Erfindung betrifft die Verwendung eines der Enzyme Pankreatin, Bromelain, Papain, Lipase, Amylase, Trypsin und/oder Chymotrypsin zur Aktivierung von Makrophagen und/oder NK-Zellen, enzymatisch aktivierte Makrophagen und/oder NK-Zellen, sowie ein Arzneimittel zur Aktivierung von Makrophagen und/oder NK-Zellen.

EP 0 421 021 A1

## VERWENDUNG KATABOLISCHER ENZYME ZUR AKTIVIERUNG VON MAKROPHAGEN UND/ODER NK-ZELLEN, EIN DIESE ENZYME ENTHALTENDES ARZNEIMITTEL SOWIE DURCH DIESE ENZYME AKTIVIERTE MAKROPHAGE UND/ODER NK-ZELLE

Die vorliegende Erfindung betrifft die Verwendung der Enzyme Pankreatin, Bromelain, Papain, Lipase, Amylase, Trypsin und/oder Chymotrypsin zur Aktivierung von Makrophagen und/oder NK-Zellen, enzymatisch aktivierte Makrophagen und/oder NK-Zellen sowie ein Arzneimittel zur Aktivierung von Makrophagen und/oder NK-Zellen.

Als Makrophagen werden mononukleäre Phagozyten (Monozyten) bezeichnet, die zu den phagozytierenden Leukozyten gehören.

NK ("natural killer")-Zellen stellen eine Subpopulation der Lymphozyten dar.

Makrophagen und NK-Zellen kommen eine wichtige Funktion der immunologischen Überwachung sowohl gegenüber Tumorzellen als auch gegenüber Infektionen zu. So ist schon länger bekannt, daß aktivierte Makrophagen und NK-Zellen auf unspezifische Weise in vitro und in vivo eine ganze Reihe von verschiedenen Tumorzellen abtöten können. Die Aktivierung der tumoriziden Effektorzellen (Makrophagen, NK-Zellen) kann mit Lymphokinen, wie Interferon und anderen Immunstimulantien erhöht werden. Interferone verden in aufwendigen und teuren Verfahren entweder aus Zellkulturen isoliert oder mit Hilfe von gentechnologischen Methoden synthetisiert.

Der vorliegenden Erfindung liegt die Aufgabe zugrunde, einen kostengünstigen, wirksamen Aktivator des tumoriziden Potentials von Effektorzellen (Makrophagen, NK-Zellen) sowie enzymatisch aktivierte Effektorzellen und ein Arzneimittel zur Aktivierung von Effektorzellen zur Verfügung zu stellen.

Diese Aufgabe wird erfindungsgemäß dadurch gelöst, daß zur Aktivierung von Makrophagen und/oder NK-Zellen mindestens eines der Enzyme Pankreatin, Bromelain, Papain, Lipase, Amylase, Trypsin und/oder Chymotrypsin verwendet wird.

Es hat sich überraschenderweise gezeigt, daß die Enzyme Pankreatin, Bromelain, Papain, Lipase, Amylase, Trypsin und/oder Chymotrypsin Makrophagen und/oder NK-Zellen hocheffizient und in extrem kurzer Zeit (innerhalb von 5 - 10 Minuten) aktivieren können.

Die erfindungsgemäß verwendeten Enzyme lassen sich kostengünstig aus den folgenden Rohmaterialien isolieren.

Pankreatin wird aus Schweine- oder Rinderpankreas gewonnen.

Bromelain ist ein proteolytisch wirksames Enyzm aus dem Preßsaft der Ananas.

Papain ist ein proteolytisches Enzym, das aus dem Milchsaft der unreifen, fleischigen Früchte des Melonenbaums Carica papaya gewonnen wird.

Lipasen gehören zu der Untergruppe der Esterasen und werden aus Pankreas oder dem Pilz Rhizopus arrhizus gewonnen.

Amylasen sind Glykosid-spaltende Enzyme, die beispielsweise aus Pankreas oder speziellen Mikroorganismen isoliert werden.

Trypsin und Chymotrypsin sind proteolytische Enzyme, die ebenfalls im Pankreas gebildet werden und in Verbindung mit anderen Enzymen bereits therapeutisch eingesetzt wurden.

Vorzugsweise wird als Lipase Triacylglycerollipase und/oder als Amylase $\alpha$-Amylase verwendet. Diese zeigen eine gute Wirkung als Aktivator des tumoriziden Potentials von Makrophagen und/oder NK-Zellen.

Eine besondere Wirksamkeit zeigt sich bei Verwendung einer Kombination der Enzyme Pankreatin, Bromelain, Papain, Triacylglycerollipase, $\alpha$-Amylase, Trypsin und/oder Chymotrypsin. Neben der bemerkenswerten und unerwarteten Wirkung dieser Enzyme auf die Aktivierung des tumoriziden Potentials von Makrophagen und/oder NK-Zellen zeigt die kombinierte Verwendung der genannten Enzyme weiterhin einen synergistischen Effekt.

Weiterhin kann vorzugsweise zusätzlich Rutosid, ein zu den Flavonoiden gehörendes Glykosid, verwendet werden.

Eine besonders gute Verträglichkeit und Wirksamkeit hat die kombinierte Verwendung von 50 - 200 mg, vorzugsweise 100 mg Pankreatin, 20 - 100 mg, vorzugsweise 45 mg Bromelain, 40 - 100 mg, vorzugsweise 60 mg Papain, 5 -50 mg, vorzugsweise 10 mg Triacylglycerollipase, 5 - 50 mg, vorzugsweise 10 mg $\alpha$-Amylase, 10 - 30 mg, vorzugsweise 24 mg Trypsin, 1 - 10 mg, vorzugsweise 1 mg Chymotrypsin und 10 - 100 mg, vorzugsweise 50 mg Rutosid x $3H_2O$ pro Dosiseinheit.

Weiterhin können die zu verwendenden Präparate zusätzlich Serratia-Peptidase enthalten. Serratia-Peptidase kann aus einem Mikroorganismus der Gattung Serratia gewonnen werden.

In einer besonders bevorzugten Ausführungsform wird zusätzlich noch Polyethylenglykol (PEG) und/oder Polyvinylpyrrolidon (PVP) verwendet. Hierdurch läßt sich die Aktivität von Effektorzellen zusätzlich

2

steigern.

Das zur Anwendung kommende Präparat kann weiterhin übliche Hilfs- und/oder Trägerstoffe enthalten.

Die durch Behandeln der Effektorzellen mit mindestens einem der Enzyme Pankreatin, Bromelain, Papain, Lipase, Amylase, Trypsin und/oder Chymotrypsin erhältlichen enzymatisch aktivierten Makrophagen und/oder NK-Zellen können zur Behandlung von Tumoren in den Patienten injiziert werden. Es hat sich nämlich überraschenderweise gezeigt, daß der enzymatisch aktivierte Zustand der Effektorzellen nach Einfrieren und späterem Auftauen derselben erhalten bleibt. Es ist hierbei von Vorteil, wenn die enzymatisch behandelten Effektorzellen vor dem Einfrieren oder der Injizierung mindestens 20 Minuten, bevorzugt mindestens 30 Minuten regenerieren können.

Die Beispiele erläutern die Erfindung.


I. Aktivierung von Makrophagen

Bei der Untersuchung der Wirkung von Enzymen und Enzymgemischen auf die Aktivität von Makrophagen wurden die folgenden Parameter bestimmt:

a) (PR %) Relative Phagozytoserate (in %) bezeichnet den in der gewählten, standardisierten Versuchsanordnung von Makrophagen/Monozyten phagozytierten prozentuellen Anteil der vorgelegten Zielzellen, d.h. der $^{51}$Cr-beladenen Schafserythrozyten (SRBC).

Die Berechnung erfolgt im $^{51}$Cr-Phagozytosetest - in weitgehender Analogie zur Berechnung der spezifischen Lyse im $^{51}$Cr-Release-Cytotoxizitätstest - nach der Formel:

Relative Phagozytoserate (in %) =

$$\frac{cpm(Probe) - cpm(negative\ Kontrolle)}{cpm(total) - cpm(Leerwert)}$$

b) (dPR %) Relative Erhöhung der Phagozytoserate (in %) bezeichnet die prozentuelle Steigerung der Phagozytoserate durch Enzyme vorbehandelter gegenüber unbehandelten Monozyten/Makrophagen.

c) (PI) Phagozytoseindex bezeichnet den pro 100 Monozyten/Makrophagen phagozytierten Anteil von Zielzellen ($^{51}$Cr-markierten SRBC) innerhalb einer Probe (Vertiefung in der 96er Mikrotitrationsplatte).

Zur Berechnung des Phagozytoseindex muß neben der Phagozytoserate die Anzahl der SRBC in einer Probenvertiefung und die Zahl der Monozyten/Makrophagen in dieser Vertiefung oder zumindest das Verhältnis von SRBC von Monozyten/Makrophagen, d.h. von Target (T)- und Effektor (E)-Zellen bekannt sein. Dann gilt:

$$PI = \frac{T}{E} \times PR\ \% \times 100 =$$

$$\frac{Zahl\ der\ SRBC\ je\ Vertiefung}{Zahl\ der\ Makrophagen\ je\ Vertiefung} \times PR\ \% \times 100$$

d) (dPI) Veränderung des Phagozytoseindex bezeichnet die Anzahl von SRBC einer Probe, die pro 100 Monozyten im Durchschnitt mehr (oder weniger) phagozytiert werden, wenn die Monozyten mit Enzymen vorbehandelt waren, gemessen an derjenigen von 100 Monozyten phagozytierten SRBC-Anzahl im Basismedium (ohne Enzym).

dPI = PI(vorbehandelt) - PI (unbehandelt)

e) (f) Faktor der Phagozytoseänderung bezeichnet den Quotienten der Phagozytoseraten vorbehandelter und unbehandelter Proben.

3

$$f = \frac{PR\ \%\ (vorbehandelt)}{PR\ \%\ (unbehandelt)} =$$

$$\frac{cpm\ (vorbehandelt) - cpm\ (negative\ Kontrolle)}{cpm\ (unbehandelt) - cpm\ (negative\ Kontrolle)}$$

1. Aktivierung von Makrophagen durch ein Enzymgemisch (Wobenzym)

Wobenzym ist ein Enzymgemisch mit der folgenden Zusammensetzung (Gewichtsteile) Pankreatin (100), Bromelain (45), Papain (60), Triacylglycerollipase (10), $\alpha$-Amylase (10), Trypsin (24) und Chymotrypsin (1). Zusätzlich enthält das Enzymgemisch noch 50 Gewichtsteile Rutosid x $3H_2O$.

a) Zehnfache Plasmakonzentratlon (10 x $C_{Pl}$

Versuchsreihe I (Versuchsbedingungen: Erholungszeit: 30 min, Enzymeinwirkungsdauer: 10 min, Vorzentrifugation (VZ) : 3 min/50 g, E : T-Verhältnis: 1:1-20)
Der mittlere PR %-Wert lag bei Proben ohne Enzymbehandlung bei 10,3 und bei Proben mit enzymatischer Vorbehandlung bei 14,7. Die entsprechenden PI-Werte lagen bei 92,5 und 129,4. Somit brachte die Makrophagen-Vorbehandlung mit Wobenzym eine durchschnittliche Verbesserung des dPI-Wertes um 36,9. Der f-Wert betrug 2,21.
Versuchsreihe II (Versuchsbedingungen: bei Versuchsreihe I, nur E : T = 1 : 20-40)
Hier lag der PR %-Wert unbehandelter Proben bei 2,8 und jener der enzym-vorbehandelten Proben bei 7,6. Entsprechende PI-Werte waren 95,2 und 258,4. Dementsprechend erhöhte sich der dPI-Wert um 163,2. Der f-Wert lag bei 2,75.
Versuchsreihe III (Versuchsbedingungen: wie bei Versuchsreihe I, nur E: T = 1:40)
Die unbehandelten Proben wiesen einen PR %-Faktor von 2,4, die enzym-behandelten einen von 4,6 auf, die entsprechenden PI-Werte errechneten sich zu 161,7 bzw. 282,9. Somit brachte die Wobenzym-Behandlung einen dPI-Anstieg um 121,2. Der f-Wert machte im Schnitt 1,75 aus.

b) Einfache Plasmakonzentration ($C_{Pl}$)

Versuchsreihe I (Versuchsbedingungen: Erholungszeit: 30 min, Enzymeinwirkungsdauer: 10 min, Vorzentrifugation: 5 min/ 50 g - 3 min/80 g, E : T-Verhältnis: 1:1 - 1:40, tz.v = 90)
Der PR %-Wert betrug nach 6maliger Wiederholung des gleichen Versuches im Durchschnitt 4,4 im Vergleich zu 2,5 in Abwesenheit von Enzymen. Die entsprechenden PI-Werte lagen bei 138,8 bzw. 111,3. Somit führte die Vorbehandlung der Makrophagen mit Wobenzym zu einem durchschnittlichen dPI-Anstieg um 27,5.
Versuchsreihe II (Versuchsbedingungen: wie bei Versuchsreihe I, nur tz.v = 150)
Die 7mal wiederholten Versuche ergaben bei Wobenzymvorbehandelten Makrophagen einen mittleren PR %-Wert von 13,2 und einen PI-Mittelwert von 390,6. Die entsprechenden Mittelwerte unbehandelter Proben betrugen 10,8 und 271,0. Die durchschnittliche dPI-Zunahme nach der Enzymbehandlung lag bei 119,6, der f-Index bei 1,53.
Versuchsreihe III (Versuchsbedingungen: wie bei Versuchsreihe I, nur tz.v = 250)
Das Ergebnis auf fünf Einzelversuchen ergab, daß Wobenzym den dPI-Index um durchschnittlich 17,4 steigen ließ. Die PI-Werte unbehandelter Proben lagen im Schnitt bei 524,5 und bei enzymatisch vorbehandelten Proben bei 541,9. Die entsprechenden PR %-Werte betrugen 19,0 und 19,7.
Die Aktivität der Makrophagen konnte zusätzlich gesteigert werden, wenn dem Kulturmedium neben Wobenzym noch PEG und/oder PVP zugesetzt wurden.

4

Versuchsreihe IV

In einer aus 6 Einzelversuchen bestehenden Versuchsserie konnte eine durchschnittliche dPI-Zunahme von 148,9 als Resultante des PI-Anstiegs von 134,5 auf 283,4 nach kombinierter Wobenzym/PEG-PVP-Vorbehandlung bebachtet werden. Der PR %-Wert stieg nach der kombinierten Wobenzym/PEG-PVP-Vorbehandlung von 3,8 auf 7,9.

Versuchsreihe V

Nach einer aus 9 Einzelversuchen bestehenden Versuchsserie, bei der Makrophagen teils mit Wobenzym allein, teils mit der Kombination von PEG-PVP und Wobenzym vorbehandelt worden, nahm der dPI-Wert um durchschnittlich 108,7 zu.

Versuchsreihe VI

In einer weiteren aus 18 Einzelversuchen zusammengesetzten Versuchsserie konnte die Makrophagen-Vorbehandlung mit Proteasen und Lipasen des Enzympräparates Wobenzym ($C_{PI}$) eine durchschnittliche dPI-Zunahme um 60,2 erwirken. Der PI-Mittelwert für die unbehandelten Proben lag bei 288,2 und jene für enzymbehandelte bei 348,4. Die entsprechenden PR %-Werte betrugen 12,8 und 9,9.

Die statistische Bearbeitung der PR %-, der PI-, der dPI und der f-Werte, gewonnen in 48 Einzelversuchen mit je 3-4, in einigen Fällen 2 Parallelen, ergab, daß die Vorbehandlung der Monozyten/Makrophagen mit Wobenzym allein ($C_{PI}$) oder mit der Kombination von Wobenzym ($C_{PI}$) und PEG-PVP (2-20 g/l PEG mit MG 6kD bzw. 1-10 g/l PVP mit MG 40KD bzw. $1 \times 10^{-3} - 1 \times 10^{-4}$ g/l PVP mit MG 360kD) zum durchschnittlichen PR %-Anstieg von 8,4 (bei unbehandelten Proben) auf 11,5 (nach Makrophagenbehandlung) führt. Die ensprechenden PI-Mittelwerte betrugen 238,1 (bei unbehandelten Proben) bzw. 319,8 (bei vorbehandelten Proben). Der dPI-Wert lag im Durchschnitt bei 81,5, der f-Wert lag bei 1,90.

2. Die Effizienz von Hydrolasen (Wobenzym) in kombinierter Verwendung mit PEG und PVP

In je 2 parallelen Versuchsserien (Versuchsreihen XI - XIV) wurde unter ansonsten konstanten Versuchsbedingungen die Effizienz der Makrophagen-Vorbehandlung mit Wobenzym allein ($C_{PI}$) jener nach kombinierter, d.h. PEG-PVP-unterstützter, enzymatischer Makrophagen-Vorbehandlung gegenüber gestellt.

Die ersten 2 parallelen Versuchsserien (Versuchsreihen XI und XII) unterschieden sich von den beiden anderen (Versuchreihen XIII und XIV) nur darin, daß tz.v im ersteren Fall 150 und im letzteren Fall 250 betrug. Die übrigen Versuchsbedingungen waren konstant (E-T-Vorzentrifugierung: 5 min/50 g, Regenerierungszeit: 30 min).

Ein direkter Vergleich der PR %-, der PI- und der dPI-Werte zwischen Versuchsreihe XI und XII (tz.v = 150) sowie zwischen Versuchsreihe XIII und XIV (tz.v = 250) vermittelt ein Bild über die potenzierende Wirkung von PEG und PVP auf die Monozyten/Makrophagen-Aktivierung durch Hydrolasen (Wobenzym)

So stieg der durchsohnittliche PI-Wert bei alleiniger Wobenzymbehandlung von 271,0 (unbehandelt) auf 390,6 (behandelt), entsprechend einem dPI-Wert von 119,6, während der entsprechende PI-Wert bei kombinierter Makrophagen-Vorbehandlung von 224,4 auf 517,0 "sprang", was einem dPI-Wert von 292,6 entspricht.

Der PR %-Wert nahm bei alleiniger Wobenzymbehandlung von 10,8 (unbehandelt) bis 15,2 (behandelt) zu; bei kombinierter Vorbehandlung verändert er sich von 5,1 auf 11,7.

Diese Ergebnisse beziehen sich auf tz.v = 150 (Versuchsreihen XI und XII). Bei tz.v = 250 (Versuchsreihen XIII und IX) fielder Vergleich zwischen einfacher und kombinierter Wobenzym-Vorbehandlung wie folgt aus:

Der durchschnittliche PI-Wertstieg bei alleiniger Wobenzymbehandlung von 524,5 (unbehandelt) auf 514,9 (behandelt) an, entsprechend einem dPI-Wert von 17,3. Bei der mit PEG- bzw. PVP-kombinierten Vorbehandlung dagegen nahm der dPI-Wert um 134,8 Einheiten (von 173,2 auf 308,1) zu. Die PR %-Werte stiegen in Abwesenheit von PEG bzw. PVP von 19,0 - 19,7 und in deren Anwesenheit von 6,4 - 11,5 an.

3. Aktivierung der Makrophagen durch Trypsin ($C_{pI}$)

Die Versuchsbedingungen entsprachen jenem bei Wobenzym (Erholungszeit: 30 min, Enzymeinwirkungsdauer: 10 min, Vorzentrifugation: 3 min/50 g). Variiert wurde in erster Linie das Verhältnis zwischen Effektorzellen (Makrophagen) und Zielzellen ($^{51}$Cr-beladenen SRBC), d.h das E : T-Verhältnis.

## 4. E : T-Verhältnis: 1:1-10

Der PR %-Wert, d.h. die "relative Phagozytoserate" (in %) veränderte sich im Schnitt von 11,1 auf 13,4 als Folge der Makrophagen-Vorbehandlung mit Trypsin ($Cl_{PI}$).

Der PI-Wert, d.h. die pro 100 Monozyten insgesamt phagozytierte Anzahl an SRBC innerhalb einer Probe, nahm in gleicher Zeit von 92,5 auf 111,9 zu. Der dPI-Wert stieg somit um 19,5 Einheiten. Der Faktor f, entsprechend dem Quotienten aus der Phagozytoserate der behandelten und unbehandelten Probe, lag bei 1,43.

## E : T-Verhältnis: 1:10-20

Die Trypsinbehandlung der Makrophagen führte zum Anstieg der "relativen Phagozytoserate" (PR %) von 17,9 auf 24,2 und zur Zunahme des Phagozytose-Index (PI) von 233,3 (unbehandelt) auf 316,1 (behandelt).

Der dPI-Wert wies einen Mittelwert von 82,8 und der Phagozytoseänderungsfaktor f einen solchen von 1,32 auf.

## E : T-Verhältnis: 1:40-80

Dieser 7 Einzelversuche mit 26 Parallelen umfaßende Komplex zeigte folgende Tendenzen: PR % stieg von 4,76 auf 5,3 und PI von 204,9 auf 225,5 an. Der Phagozytose-Index veränderte sich um 20,6 und der Faktor f wies im Schnitt einen Wert von 1,24 auf.

## E : T-Verhältnis: 1:80

Der 4mal (mit 15 Parallelen) durchgeführte Versuch zeigte ähnlichen Trend wie die Versuchsreihen zuvor: PR % stieg mäßig von 3,1 auf 3,7 nach Trypsin-Behandlung. In gleicher Zeit erhöhte sich der Phagozytose-Index von 275,1 (ohne Vorbehandlung) auf 325,5. Der Faktor f betrug 1,19, der mittlere dPI-Index 50,4.

## E : T-Verhältnis: 1:1-80

Die Zusammenfassung von 18 Versuchen (mit 68 Parallelen) ohne nähere Spezifikation des E : T-Verhältnisses resultiert in einem Anstieg des PR %-Mittelwertes von 8,0 (vor Behandlung) auf 9,9 (nach Behandlung), in einer PI-Zunahme von 200,3 auf 237,6 sowie in einem Mittelwert für dPI von 37,2 sowie für f von 1,28.

## 5. Einhaltung einer obligaten Regenerierungszeit von wenigstens 20, bevorzugt 30 Minuten bei der in vitro Aktivierung von Makrophagen durch Hydrolasen, demonstriert am Beispiel des Trypsins ($C_{PI}$))

Im folgenden Versuch wurde die Einhaltung bzw. Nicht-Einhaltung einer Regenerierungszeit von 30 min nach Enzymbehandlung untersucht. Wurde keine Regenerierungszeit eingehalten, so fiel die mittlere "relative Phagozytoserate" (PR %) von 8,3 auf 7,9. Auch der gemittelte PI nahm von 252,7 auf 219,2 ab. Somit fiel der dPI-Index negativ aus (-33,5) und der f-Faktor lag bei 0,86. Wenn man allerdings unter sonst gleichen Versuchsbedingungen eine Regenerierungszeit von 30 min einhielt, ergab sich dagegen eine Änderung der PR % nach Trypsinbehandlung von 7,9 auf 9,9, das IP von 200,2 auf 237,6, dPI zeigte einen Mittelwert von 37,3 und der f-Faktor lag bei 1,28.

## 6. Makrophagen-Aktivierung durch Papain

Es wurden zwei Papain-Konzentrationen getestet, die $C_{PI}$ und ihr 10faches. Der ersten Versuchsserie wurde Papain in der $C_{PI}$-Konzentration untersucht. Die Papain-vorbehandelten Makrophagen wiesen einen PR %-Mittelwert von 11,3 und einen PI-Mittelwert von 165,9 auf. Die unbehandelten Makrophagen zeigten dagegen einen niedrigeren PR %-Mittelwert von 7,5 und einen PI-Mittelwert von 164,6.

Die 10fach höhere Papain-Konzentration bewirkte einen mittleren PR %-Anstieg von 2,9 auf 5,4 und eine PI-Verschiebung von 139,0 auf 265,3. Der dPI-Mittelwert lag bei 196,2 und der f-Mittelwert bei 1,85.

## 7. Aktivierung der Effektorzellen (Makrophagen) durch Lipase

Die Versuchsbedingungen waren gleich wie bei den anderen Enzymen. In einer Versuchsserie, bestehend aus 8 Einzelversuchen (mit 29 Parallelen), ergab die Vorbehandlung der Makrophagen mit der therapeutisch erzielbaren Lipase-Konzentration ($C_{PI}$) eine Steigerung der phagozytischen Aktivität von Makrophagen, die sich in folgenden (gemittelten) Kennzahlen äußerte: Die relative Phagozytoserate (PR %) stieg von 10,3 bei unbehandelten auf 12,3 bei Lipase-vorbehandelten Proben an. Der Phagozytoseindex (PI) erhöhte sich von 258,8 auf 316,8 Einheiten. Der dPI-Mittelwert errechnete sich zu 58,0 und der f-Mittelwert lag im Durchschnitt bei 1,24.

In einer weiteren Serie von 8 Einzelversuchen, bei denen die Lipase zum Teil auch mit PEG (MG 6kD, 40 g/l) und PVP (MG 360kD, 0,1 - 10 mg/l) kombiniert wurde, erhöhte sich die relative Phagozytoserate nach der Enzymbehandlung von 16,6 auf 20,4 und der Phagozytose-Index von 329,9 auf 411,2. Der entsprechende dPI-Mittelwert lag bei 59,0 und der gemittelte f-Wert bei 1,24.

Die zusammenfassende Darstellung von 26 Einzelversuchen (mit 76 Parallelen) ergab im Durchschnitt eine PR %-Steigerung von 10,1 auf 12,8 (im Extremfall von 3,0 auf 13,2) und eine durchschnittliche PI-Zunahme von 230,1 auf 296,3 (im Extremfall von 75,0 auf 330,0). Der dPI-Mittelwert lag bei 66,3 (Höchstwert: 255,0) und der f-Mittelwert bei 1,35 (Höchstwert: 4,37).

## 8. Synergismus der einzelnen Hydrolasen (Proteasen und Lipasen)

Überraschenderweise wurde eine synergistischen Wirkung des Hydrolasen-Gemisches Wobenzym gefunden. Im folgenden wurde die Wirkung einzelner Vertreter der tierischen und pflanzlichen Proteasen sowie Lipase der Wirkung des Enzympräparates gegenübergestellt.

### a) Vergleich von Trypsin ($C_{PI}$) mit dem Enzympräparat ($C_{PI}$)

In einer 13 Einzelversuche umfaßenden Versuchsserie zeigte sich, daß der durchschnittliche PR %-Wert bei Wobenzym (10,2) um 1,8 höher als bei Trypsin (8,4) lag bzw. deren Verhältnis 1,2 betrug.

Der PI-Index war bei Wobenyzm (274,0) um 49,7 Einheiten höher als bei Trypsin (224,3). Im Extremfall differierten die PR %-Werte um 8,1 und der PI-Index um 281,6 Einheiten zu Gunsten des Wobenzyms.

### b) Vergleich von Chymotrypsin mit dem Enzymgemisch

Eine auf 9 Einzelversuchen aufbauende Versuchsserie führte zu folgendem Ergebnis:
Der Mittelwert der "relativen Phagozytoserate" lag beim Wobenzym (12,2) um 2,4 Einheiten höher als beim Chymotrypsin (9,8); der "Phagozytose-Index" wies beim Wobenzym (264,0) einen 24,8 Einheiten höheren Mittelwert als beim Chymotrypsin (239,2) auf. Das Verhältnis beider PR %-Werte lag bei 1,26 zu Gunsten des Wobenzyms.

Im Extremfall unterschieden sich die PR %-Werte um 13,1 und die PI-Werte gar um 170,3 zu Gunsten des Enzymgemisches.

### c) Vergleich von Papain mit dem Enzymgemisch

Auch bei diesem Vergleich wies Wobenzym eine höhere PR %-Aktivität als Papain auf (19,5 versus 14,8)

Auch die PI-Werte lagen bei Wobenzym höher (311,2 versus 269,0).

## d) Vergleich der Lipase mit dem Enzymgemisch

In einer 5 Einzelversuche umfaßenden Vergleichsserie wies Wobenzym mit einem PR %-Mittelwert von 11,5 eine um 2,5 Einheiten höhere phagozytose-steigernde Aktivität als Lipase (9,0) auf. Ähnliches gilt für die PI-Mittelwerte (292,3 versus 275,6).

Die gleichzeitige Untersuchung von (a) Trypsin (b) Chymotrypsin und (c) Wobenzym (alle: $C_{PI}$)) in einer aus 9 Einzelversuchen bestehenden Vergleichsserie resultierte in folgenden Mittelwert für die aktivitätsrelevanten Kennzahlen PR %, PI und f:

(a) Trypsin: PR % 10,2, PI 215,1 und f 1,37

(b) Chymotrypsin: PR % 9,9, PI 239,2 und f 1,46 sowie

(c) Wobenzym: PR % 12,2, PI 264,0 und f 1,74.

Die entsprechenden PR %- und PI-Mittelwerte der unbehandelten Referenzproben betrugen 7,9 und 171,9 (f 1,0).

## II. Steigerung der Aktivität von NK-Zellen durch Hydrolasen (proteolytische Enzyme tierischen und pflanzlichen Ursprungs, Lipase)

Die cytotoxische/cytolytische Aktivität von NK-Zellen wurde im $^{51}$Cr-Release-Test quantitativ erfaßt. Als Zielzellen (T) dienten K562-Lymphomzellen, die besonders empfindlich und gewissermaßen selektiv für (aktivierte) NK-Zellen sind.

Im folgenden werden die gemessenen Parameter erläutert:

a) (LR %) Lyserate bezeichnet mathematisch die prozentuale Zytotoxizität und wird nach der folgenden Formel berechnet:

$$LR = \frac{cpm(Probe) - cpm(negative\ Kontrolle)}{cpm(total) - cpm(negative\ Kontrolle)} \times 100\ (\%)$$

b) (LI %) Lyseindex bezeichnet die Anzahl der K562-Zellen, die von je 100 NK-Zellen lysiert werden in %.

$$LI = \frac{Anzahl\ der\ K653 - Zellen\ (Probe)}{Anzahl\ der\ NK-Zellen\ (Probe)} \times Lyserate(in\ \%) \times 100$$

c) (dLR) Änderung der Lyserate bezeichnet die Differenz der Lyseraten (LR) zwischen Enzym-behandelten und nicht behandelten Proben. Der Referenzwert ist demnach die NK-Aktivität im Basismedium, d.h. ohne Enzym und/oder PEG-PVP-Zusatz.

## 1. NK-Aktivierung unter Einhaltung einer Regenerierungszeit

Die Vorbehandlung der NK-Zellen (10 min bei 37°C) mit dem Enzymgemisch (Wobenzym) bei der therapeutisch erzielbaren Plasmakonzentration ($C_{PI}$) zeigte zwar bei verschiedenen Testpersonen stark differierende Absolutwerte für die "Lyserate" (LR), für den Lyseindex (LI) sowie für die Änderung der Lyserate nach Enzymbehandlung (dLR), der Trend zur enzym-vermittelten NK-Aktivierung ist jedoch klar ausgeprägt.

In einer aus 26 Einzelversuchen bestehenden Versuchsserie zeigte sich nur in einem Fall eine Aktivitätsabnahme nach Wobenzym-Behandlung.

Um ein genaueres Bild vor allem auch hinsichtlich der beachtlichen interindividuellen Schwankungen zu vermitteln, seien die LR-, die LI- und die dLR-Werte dieser repräsentativen Versuchsserie einzeln aufgeführt. So änderte sich der LR-Wert von Proband zu Proband entsprechend den Zahlenpaaren links und rechts des Trennstrichs (links: unbehandelte Proben, rechts: enzym-vorbehandelte Proben): 3,0/3,5; 3,0/4,9; 3,1/20,0; 3,1/47,4; 6,2/8,0; 6,2/8,9; 12,3/13,4; 12,3/36,1; 14,1/16,5; 14,1/42,4; 2,9/11,0; 8,5/12,1; 10,4/19,3; 20,8/21,3; 30,2/44,6; 25,2/26,5; 0,9/12,6; 5,7/31,3; 7,8/25,2; 18,4/25,5; 25,7/37,0; 25,1/25,7; 19,2/17,7; 22,1/23,6; 23,7/24,1; 23,1/24,3.

Die entsprechenden LI-Werte änderten sich von Proband zu Proband wie folgt (links: unbehandelt, rechts: enzym-vorbehandelt): 60/71; 60/98; 62/401; 62/948; 124/160; 124/178; 246/267; 246/721; 282/330; 282/849; 57/219; 170/241; 207/387; 416/425; 603/892; 503/530; 17/252; 115/626; 157/505; 368/511; 514/739; 502/513; 384/354; 442/473; 474/482; 462/485.

die hieraus berechneten dLI-Werte waren: 11; 38; 339; 886; 36; 54; 21; 475; 48; 567; 162; 71; 180; 9, 289; 27; 235; 511; 348; 143; 225; 11; 31; 8; 23.

2. Behandlung der NK-Zellen mit dem Enzymgemisch Wobenzym unter keiner Einhaltung einer Regenierungszeit

Die Werte werden bei diesen Untersuchungen meistens negativ. LR bewegte sich von 0,7/3,3 - 18,7/7,2, die Werte für LI lagen zwischen 14/67 und 431/233, für die dLI zwischen 53 und -229. Die Ergebnisse machen die Wichtigkeit einer Regenerierungszeit für die Zellen deutlich.

3. Aktivierung der NK-Zellen durch Trypsin

Trypsin erwies sich als eine der aktivsten Komponenten des Hydrolasengemisches. Um die eindeutige Wirkung, aber auch die großen interindividuellen Schwankungen, die sowohl die Basisaktivität als auch die enzym-vermittelte Aktivierbarkeit der NK-Zellen betreffen, aufzuzeigen, sollen die Ergebnisse einer aus 28 Einzelversuchen aufbauenden Versuchsserie einzeln aufgelistet werden. Wie oben bei Wobenzym sollen die LR-, die LI- und die dLI-Werte wiedergegeben werden. Auch hier steht die Zahl links vom Trennstrich für die unbehandelte und jene rechts von diesem Querstrich für enzym-vorbehandelte Proben.

Die LR-Werte waren: 7,6/12,8; 3,0/3,6; 7,6/18,8; 3,0/7,6; 3,1/32,6; 6,2/12,4; 3,1/32,6; 6,2/12,4; 12,3/25,9; 6,2/12,9; 12,3/26,4; 14,1/36,3; 17,3/17,8; 14,1/37,6; 25,4/27,1; 21,3/22,9; 22,8/23,8; 25,3/27,8; 22,6/26,6/ 42,2/26,6; 30,3/31,9; 13,0/17,5; 20,2/24,1; 20,6/26,4; 24,5/21,4; 30,4/32,3; 23,3/27,1; 27,5/30,9; 26,6/31,8.

Die entsprechenden LI-Werte änderten sich von Proband zu Proband wie folgt: 155/256; 60/72; 155/377; 60/152; 62/651; 124/249; 62/239; 124/259; 246/517; 282/727; 246/538; 282/752; 345/355; 508/542; 425/458; 455/476; 505/556; 452/533; 483/533; 605/637; 260/350; 404/483; 413/527; 490/429; 608/646; 456/541; 549/619; 532/635.

Die dLI-Werte nahmen folgende Werte an: 101; 12; 222; 92; 589; 125; 177; 135; 271; 445; 292; 470; 10; 34; 33; 21; 51; 81; 50; 32; 90; 79; 114; -61; 38; 76; 70; 70; 103.

4. Wirkung von Chymotrypsin auf die Aktivität von NK-Zellen

Auch bei Chymotrypsin zeigte sich die gleiche Wirkung wie bei Wobenzym und Trypsin. Es zeigte sich immmer eine Steigerung der NK-Aktivität, solange die obligate Erholungszeit unmittelbar nach der Enzymbehandlung eingehalten wurde.

Bei einem typischen Experiment, das auf 6 Einzelversuchen aufbaute, bewegten sich die Wertepaare für die "Lyserate" (LR) zwischen 6,2/9,2 und 3,1/17,5 jene für die "Lyserate" (LR) zwischen 124/185 und 62/349 und die dR-Werte zwischen 61 und 287.

5. Steigerung der NK-Aktivität durch Papain

Auch bei NK-Zellen, die mit der pflanzlichen Protease Papain vorbehandelt worden sind, zeigte sich eine klare Aktivitätssteigerung.

Dies soll am Beispiel einer repräsentativen Versuchsserie, die auf 8 Einzelversuchen basierte, demonstriert werden.

Die LR-Wertpaare bewegten sich von Proband zu Proband variierend von 0,7/1,9 - 14,1/45,7, die entsprechenden LI-Wertpaare lagen zwischen 14/37 und 282/915 und die dLI-Werte streuten zwischen 23 und 633.

6. Steigerung der Aktivität von NK-Zellen durch Lipase

Der Einfluß der im Wobenzym enthaltenen Lipase auf die NK-Aktivität wird am Beispiel einer aus 32 Einzelversuchen bestehenden Versuchsserie demonstriert. Es zeigte sich, daß eine klare Wirkung auf die Aktivität von NK-Zellen besteht, daß jedoch auf die interindividuellen Schwankungen sowohl der Basisaktivität als auch der Lipase-vermittelten Aktivierung von NK-Zellen beachtlich sind. Nur in 3 von 32 Fällen ergaben sich für dLI negative Werte.

Die Aufschlüsselung nach den Aktivierungskennzahlen LR, LI und dLI sowie die Schreibweise bei den LR- und LI-Wertepaaren (links: unbehandelt, rechts: Lipase-vorbehandelt) entsprechen jenen bei den oben besprochenen Enzymen.

LR ("Lyserate") : 2,2/3,1; 5,7/7,1; 4,6/7,1; 4,3/5,5; 2,4/7,3; 5,9/7,2; 7,1/7,2; 5,7/6,4; 4,3/9,6; 1,9/1,4; 5,2/6,4; 3,4/5,4; 4,7/5,2; 4,2/7,0; 5,8//,5; 6,5/6,5; 6,1/7,5; 7,0/11,0; 19,2/17,0; 22,1/23,6; 23,7/24,1; 23,1/23,4; 6,2/8,4; 6,2/7,1; 12,3/26,3; 12,3/26,0; 14,1/38,2; 14,1/39,7; 14,7/15,1; 18,7/19,4; 21,6/13,9; 19,6/20,1.

LI ("Lyseindex") : 43/62; 115/142; 93/142; 86/109; 48/147; 118/145; 143/144; 114/128; 86/191; 38/29; 105/128; 68/109; 94/104; 83/139; 116/150; 129/131; 150/122; 140/221; 384/340; 442/471; 474/481; 462/469; 124/167; 124/142; 246/527; 246/521; 282/763; 282/793; 301/293; 373/387; 431/278; 401/393.

dLI ("Änderung der Lyserate") : 19; 27; 49; 23; 99; 27; 1; 14; 105; -9; 23; 41; 10; 56; 34; 2; 28; 81; -44; 29; 7; 43; 18; 281; 275; 481; 511; 8; 14; -153; 8.

III. Um die erhaltenen, in vitro enzymatisch aktivierten Effektorzellen in Tumorpatienten zu injizieren ist die Stabilität des tumoriziden Zustandes dieser Zellen von besonderer Wichtigkeit. Es wurde überraschenderweise gefunden, daß der durch enzymatische Behandlung erzielbare, aktivierte, tumorizide Zustand der Effektorzellen nach Einfrieren und späterem Auftauen derselben erhalten bleibt.

Zum Einfrieren der enzymatisch aktivierten Effektorzellen wurden die beiden Polymere PEG und PVP als Kryoprotektanten verwendet. In diesen Versuchen wurde das PEG mit MG 6kD sowie das PVP mit MG 40KD und 360 kD in einer Endkonstellation von 40 g/l zu einer 8 - 15 %igen DMSO-Lösung im RPM I 1640-Medium zugesetzt.

Wie aus den unten aufgührten Kennzahlen ersichtlich ist, blieb bei dieser Mischung die Enzym-induzierte Makrophagen-Aktivierung auch nach Einfrieren und Auftauen der Makrophagen erhalten.

Die Versuchsbedingungen waren: Wobenzym ($C_{PI}$), Enzymeinwirkungsdauer: 10 min, Erholungszeit: 30 min.

Die nach dem Auftauen der eingefrorenen, Enzym-vorbehandelten Makrophagen-Suspensionen gemessenen Aktivitäten zeigen klar, daß die Effektorzell-Aktivierung durch das Einfrieren der Zellen nicht verlorengeht. So zeigten die mit dem aus DMSO (8 - 15 %) und PEG (MG 6kD, 40 g/l) zusammengesetzten kryoprotektiven Gemisch eingefrorenen Proben PR %-Werte von 4,4 - 9,7, während die Referenzwerte zwischen 2,2 und 6,1 lagen (PR %-Vergleichswerte: 9,7/2,2, 4,4/2,6, 8,6/6,1, 6,9/2,8) . Die PI-Werte bewegten sich zwischen 132,0 und 291,0, die Referenzwerte zwischen 66,0 und 146,4 (PI-Werte: 291,0/66,0, 132,0/78,0, 206,4/146,4, 200,1/81,2).

Die entsprechenden dPR-Werte lagen zwischen 41,0 und 340,7, die dPI-Werte zwischen 54,0 und 225,0. Die gute Konservierung der Makrophagen-Aktivität zeigte sich auch im f-Wert der zwischen 1,41 und 4,41 lag. Auf die Kombination des konventionellen Kryoprotektants DMSO mit PVP erwies sich als sehr erfolgreich bei der Konservierung der Makrophagen-Aktivität, wie sich aus dem Vergleich der PR %-Wertepaare 5,9/2,8 und 8,7/2,1 bei PVP mit MG 40kD bzw. 4,2/1,8 und 12,0/6,6 bei PVP mit MG 360 kD abgeleitet werden kann.

Das gleiche Ergebnis zeigt sich auch bei den PI-Wertepaaren (141,6/67,2 und 60,9/252,3 bei MG 40kD bzw. 196,0/54,0 und 300,0/165,0 bei MG 360kD), sowie bei den f-Werten (2,12 und 4,15 bei MG 40kD bzw. 2,34 und 1,80 bei MG 360kD)

**Ansprüche**

1. Verwendung mindestens eines der Enzyme Pankreatin, Bromelain, Papain, Lipase, Amylase, Trypsin und/oder Chymotrypsin zur Aktivierung von Makrophagen und/oder NK-Zellen.
2. Verwendung nach Anspruch 1,
**dadurch gekennzeichnet,**

daß als Lipase Triacylglycerollipase verwendet wird.

3. Verwendung nach Anspruch 1,
**dadurch gekennzeichnet,**
daß als Amylase α-Amylase verwendet wird.

4. Verwendung nach Anspruch 1,
**dadurch gekennzeichnet,**
daß eine Kombination der Enzyme Pankreatin, Bromelain, Papain, Triacylglycerollipase, α-Amylase, Trypsin und/oder Chymotrypsin verwendet wird.

5. Verwendung nach mindestens einem der Ansprüche 1-4,
**dadurch gekennzeichnet,**
daß zusätzlich Rutosid verwendet wird.

6. Verwendung nach Anspruch 5,
**dadurch gekennzeichnet,**
daß 50 - 200 mg, vorzugsweise 100 mg Pankreatin, 20 -100 mg, vorzugsweise 45 mg Bromelain, 40 - 100 mg, vorzugsweise 60 mg Papain, 5 - 50 mg, vorzugsweise 10 mg Triacylglycerollipase, 5 - 50 mg, vorzugsweise 10 mg α-Amylase, 10 - 30 mg, vorzugsweise 24 mg Trypsin, 1 -10 mg, vorzugsweise 1 mg Chymotrypsin und 10 - 100 mg, vorzugsweise 50 mg Rutosid x $3H_2O$ pro Dosiseinheit verwendet werden.

7. Verwendung nach mindestens einem der Ansprüche 1-6,
**dadurch gekennzeichnet,**
daß zusätzlich Serratia-Peptidase verwendet wird.

8. Verwendung nach mindestens einem der Ansprüche 1-7,
**dadurch gekennzeichnet,**
daß zusätzlich PEG und/oder PVP verwendet wird.

9. Enzymatisch aktivierte Makrophagen und/oder NK-Zellen erhältlich durch Behandeln der Zellen mit mindestens einem der Enzyme Pankreatin, Bromelain, Papain, Lipase, Amylase, Trypsin und/oder Chymotrypsin.

10. Arzneimittel zur Aktivierung von Makrophagen und/ oder NK-Zellen enthaltend mindestens eines der Enzyme nach Anspruch 1 - 8.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | ROTE LISTE, 1986, Nr. 85074, "Wobe-Mugos" --- | | A 61 K 37/54 |
| A | ROTE LISTE, 1986, Nr. 85075, "Wobe-Mugos" --- | | |
| D | ROTE LISTE, 1986, Nr. 41001, "Wobenzym" --- | | |
| D | EP-A-0 309 602 (MUCOS) ----- | | |

RECHERCHIERTE SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 30-01-1990 | TURMO Y BLANCO C.E. |